# DEMANDE DE BREVET EUROPEEN

(11) **EP 3 698 827 A1**
(43) Date de publication de la demande: **26.08.2020**
(21) Numéro de dépôt: 20305155.2
(22) Date de dépôt: 18.02.2020
(51) Int. Cl.: A61M 5/14, A61M 39/24

(54) **DISPOSITIF MULTIACCES DE RACCORDEMENT À UNE POMPE DE PERFUSION INTRAVEINEUSE ET/OU ENTÉRALE**

(30) Priorité: 20.02.2019 FR 1901707
(71) Demandeur: Cair L. G. L., 69380 Lissieu (FR)
(72) Inventeur: JACQUIN, Philippe, 38230 Chavagneux (FR)
(74) Mandataire: Cabinet Laurent & Charras

(57) **Abrégé**

L'invention concerne un dispositif multiaccès de raccordement (10a) à une pompe de perfusion (11) intraveineuse et/ou entérale, ledit dispositif comportant :
- un premier connecteur supérieur (24c) destiné à acheminer un produit de rinçage ou d'hydratation ;
- au moins un second connecteur supérieur (24a, 30) destiné à acheminer au moins un produit de traitement ;
- une tubulure inférieure (13a, 13b) destinée à être connectée à une pompe (11) d'une perfusion ;
- au moins un raccord multiaccès (17b) mettant en connexion fluidique les connecteurs supérieurs (24a, 24c, 30) et ladite tubulure inférieure (13a, 13b) ; et
- une ligne de rinçage ou d'hydratation (25a) disposée entre l'au moins un raccord multiaccès (17b) et ledit premier connecteur supérieur (24c) ;
ladite ligne de rinçage ou d'hydratation (25a) comportant une valve unidirectionnelle à seuil (27) configurée pour s'ouvrir lorsqu'une dépression est formée sur ladite tubulure inférieure (13a, 13b).

## Description

### Domaine technique

L'invention se rattache au domaine des dispositifs médicaux, en particulier à la technologie des arbres multiaccès généralement utilisés pour effectuer des perfusions dans le cadre d'une thérapie intraveineuse, entérale, osseuse ou sous-cutanée.

Au sens de l'invention, un arbre multiaccès comporte une ligne de rinçage ou d'hydratation et au moins un connecteur pour une poche remplie d'un produit de traitement, destinée à être injecté dans l'organisme d'un patient.

L'invention trouve une application particulièrement avantageuse pour l'administration parentérale d'une chimiothérapie ou l'administration entérale d'un apport nutritionnel.

### Arrière-plan technologique

Dans le cadre d'une chimiothérapie ou d'apports nutritionnels, la première étape du protocole thérapeutique consiste à injecter dans l'organisme du patient un premier produit de traitement contenu dans une poche.

Dans le cadre d'une chimiothérapie, la seconde étape consiste à effectuer le rinçage du premier produit par un produit de rinçage. Le produit de rinçage correspond généralement à un produit isotopique tel que du sérum physiologique ou glucosé. Dans le cadre d'un apport nutritionnel, la seconde étape consiste généralement à hydrater le patient par l'administration d'un produit d'hydratation. Le produit d'hydratation correspond généralement à de l'eau minérale. Pour obtenir un rinçage ou une hydratation efficace sans générer d'effets secondaires chez le patient, on recherche le plus souvent à injecter le produit de traitement et le produit de rinçage ou d'hydratation à un débit constant et similaire.

Lorsque la thérapie comporte plusieurs produits de traitement à injecter, les étapes ultérieures consistent à injecter les autres produits de traitement avec une étape de rinçage ou d'hydratation intercalée entre deux étapes d'injections de produits distincts.

Pour garantir l'efficacité du traitement, il est nécessaire d'éviter toute injection d'air dans l'organisme du patient. Pour ce faire, le produit de rinçage ou d'hydratation et les produits de traitement sont classiquement intégrés dans des poches souples préalablement purgées avant de procéder à la circulation du produit de traitement dans le dispositif.

Chaque poche souple est reliée par une tubulure à une pompe par l'intermédiaire d'un ou plusieurs raccords multiaccès. Il existe des raccords multiaccès en forme de Y destinés à relier deux ou trois tubulures. De plus, certains arbres multiaccès comportent plusieurs raccords multiaccès en forme de Y de sorte à permettre la connexion d'une pluralité de produits de traitement.

Ainsi, au sens de l'invention, un arbre multiaccès correspond à un ensemble d'un ou plusieurs raccords multiaccès et d'une ou plusieurs tubulures de sorte à mettre en connexion fluidique une pompe avec un produit de rinçage ou d'hydratation et un ou plusieurs produits de traitement.

Actuellement, pour réaliser le protocole thérapeutique décrit précédemment, l'infirmière bloque l'écoulement de toutes les tubulures connectées entre les poches souples et le ou les raccords multiaccès à l'exception de la tubulure destinée à acheminer le produit à injecter. Pour ce faire, l'infirmière utilise classiquement une pince, également appelée « *clamp* » dans la littérature anglo-saxonne.

Avant d'injecter le premier produit de traitement, l'infirmière doit également régler la pompe en indiquant le débit et la quantité de produit à injecter. Pour régler la quantité de produit à injecter, l'infirmière se réfère à la quantité de produit théoriquement présente dans la poche contenant le produit de traitement. Cependant, au cours du stockage des poches souples, il se produit un phénomène d'évaporation du liquide contenu dans les poches souples. Afin d'anticiper cette évaporation, les industriels sur-dosent classiquement la quantité de produits présente dans ces poches souples.

En pratique, peu avant la fin d'un traitement thérapeutique, l'infirmière est appelée pour préparer la ligne de rinçage ou d'hydratation. Le plus souvent, l'injection du produit de traitement n'est à ce moment-là pas terminée compte-tenu du surdosage de la quantité de produit. L'infirmière doit alors attendre pour finaliser le traitement avant de pouvoir passer à l'étape de rinçage ou d'hydratation.

Lorsque tout le produit de traitement contenu dans la poche a été injecté au patient, l'infirmière fixe une pince sur la tubulure ayant servi à injecter le produit de traitement et ouvre la ligne contenant le produit de rinçage ou d'hydratation. A l'issue de cette manipulation, le débit d'injection du liquide de rinçage ou d'hydratation ainsi que la quantité à injecter sont réglés sur la pompe de perfusion. Cette étape de traitement peut donc être particulièrement longue pour une infirmière qui doit attendre la fin de l'administration du produit de traitement pour lancer l'étape de rinçage ou d'hydratation.

Au lieu d'attendre la fin de l'administration du produit de traitement, certaines infirmières sont tentées d'augmenter le débit de pompage de la poche contenant le produit de traitement pour vider la poche plus vite au risque de nuire à la qualité du traitement. D'autres infirmières choisissent d'injecter le produit de traitement sur un temps plus long que le temps nécessaire pour injecter le contenu indiqué sur la poche en préjugeant du contenu réellement présent dans la poche. Cette manipulation risque d'entraîner une mise en sécurité de la pompe lorsque la poche est vide et que la pompe continue de fonctionner. Dans tous les cas, l'imprécision sur la lecture du volume de produit effectivement contenu dans les poches induit un risque non négligeable dans le traitement thérapeutique du patient et une augmentation du temps requis de la présence de l'infirmière auprès du patient.

Pour répondre à ce problème technique, les documents US 2007/0078431 et WO 2017/197024 décrivent une pompe reliée à deux lignes distinctes : une ligne destinée à acheminer un produit de traitement et une ligne destinée à acheminer un produit de rinçage. En plus de réaliser le pompage des produits, la pompe est également capable de changer automatiquement la source en passant de la ligne de traitement à la ligne de rinçage. Pour ce faire, tel que décrit dans le document US 2007/0078431, ce changement automatique est réalisé par un robinet pouvant connecter l'une ou l'autre des deux lignes sur une ligne principale de la pompe. Cette pièce est assez complexe à réaliser et à mettre en œuvre, ce qui complexifie grandement la structure et le coût de la pompe. En outre, ce système ne permet pas d'injecter plus d'un produit de traitement au patient alors que de plus en plus de protocoles de traitement requièrent l'utilisation de plusieurs produits de traitement. Le problème technique que se propose de résoudre l'invention est de simplifier le protocole d'injection en réduisant le temps requis de présence de l'infirmière auprès du patient tout en garantissant la qualité du traitement.

### Exposé de l'invention

Pour répondre à ce problème technique, l'invention propose d'utiliser une pompe intraveineuse et/ou entérale classique associée à une valve unidirectionnelle à seuil disposée sur la ligne de rinçage ou d'hydratation afin que la dépression formée par la pompe, lorsque la première poche est vidée, entraîne automatiquement l'ouverture de la valve unidirectionnelle à seuil et l'approvisionnement en liquide de rinçage ou d'hydratation de la pompe. Ainsi, la pompe peut assurer la transition entre l'administration du produit de traitement et l'administration du produit de rinçage ou d'hydratation sans nécessiter l'intervention d'une infirmière. Ceci représente un gain de temps considérable par rapport aux techniques existantes.

En outre, cette transition automatique permet d'effectuer tout le processus d'administration avec un débit constant en garantissant que la poche contenant le produit de traitement soit complètement vidée. L'imprécision sur la quantité de liquide présent dans la poche peut à présent être ajustée par la quantité de liquide de rinçage ou d'hydratation injectée au patient, cette quantité n'étant pas préjudiciable à la qualité du traitement réalisé.

A cet effet, selon un premier aspect, l'invention concerne un dispositif multiaccès de raccordement à une pompe de perfusion intraveineuse et/ou entérale, ledit dispositif comportant :
- un premier connecteur supérieur destiné à acheminer un produit de rinçage ou d'hydratation ;
- au moins un second connecteur supérieur destiné à acheminer au moins un produit de traitement ;
- une tubulure inférieure destinée à être connectée à une pompe de perfusion intraveineuse et/ou entérale ; et
- au moins un raccord multiaccès mettant en connexion fluidique les connecteurs supérieurs et ladite tubulure inférieure.

L'invention se caractérise en ce que le dispositif comporte également une ligne de rinçage ou d'hydratation disposée entre l'au moins un raccord multiaccès et ledit premier connecteur supérieur ;
ladite ligne de rinçage ou d'hydratation comportant une valve unidirectionnelle à seuil intégrant :
une membrane mobile entre deux positions :une position de blocage dans laquelle la circulation du liquide est bloquée dans ladite ligne de rinçage ou d'hydratation au niveau de ladite valve unidirectionnelle à seuil ; et une position de circulation dans laquelle le liquide peut s'écouler dans ladite ligne de rinçage ou d'hydratation à travers ladite valve unidirectionnelle à seuil ; et
des moyens de rappel de ladite membrane dans ladite position de blocage ;
ladite valve unidirectionnelle à seuil étant montée sur ladite ligne de rinçage ou d'hydratation de sorte qu'une dépression formée sur ladite tubulure inférieure entraîne un déplacement de ladite membrane entre ladite position de blocage et ladite position de circulation, ladite valve unidirectionnelle à seuil étant destinée à coopérer avec ladite pompe de perfusion intraveineuse et/ou entérale de sorte que la dépression formée par ladite pompe de perfusion, lorsque la première poche est vidée, entraîne automatiquement l'ouverture de ladite valve unidirectionnelle à seuil et l'approvisionnement en liquide de rinçage ou d'hydratation de ladite pompe de perfusion.

L'invention permet ainsi d'automatiser la transition entre la phase d'injection d'un produit de traitement et la phase d'injection d'un produit de rinçage ou d'hydratation, tout en garantissant un débit constant d'administration des deux produits. Il n'est donc plus nécessaire à une infirmière de venir auprès du patient pour finir l'injection du produit de traitement avant l'administration du produit de rinçage ou d'hydratation. De plus, l'infirmière n'a pas à attendre la fin de l'administration du produit de traitement. Le temps de travail des infirmières peut donc être grandement optimisé ce qui contribue à une plus grande efficacité dans le traitement des patients en hôpital ou en clinique. En outre, en minimisant l'intervention d'un professionnel de la santé, il est possible de limiter son exposition aux produits de traitement qui présentent des risques toxiques non négligeables sur un organisme sain.

De préférence, ladite valve unidirectionnelle à seuil présente un seuil d'ouverture compris entre 100 et 400 mBar, préférentiellement compris entre 150 et 250 mBar. En effet, si le seuil d'ouverture de la valve est trop faible, la valve risque de s'ouvrir lors de l'administration du produit de traitement. Au contraire, si le seuil d'ouverture est trop élevé, la pompe risque de se mettre en sécurité lorsque la poche contenant le produit de traitement est vidée sans que la dépression de la pompe n'ait suffit à ouvrir la valve unidirectionnelle à seuil.

Selon un mode de réalisation, ladite ligne de rinçage ou d'hydratation comporte deux raccords multiaccès disposés au-dessus et en-dessous de ladite valve unidirectionnelle à seuil et reliés par une tubulure formant une ligne de contournement de ladite valve unidirectionnelle à seuil. Lorsque cette ligne de contournement est ouverte, la ligne de rinçage ou d'hydratation fonctionne de manière analogue à son fonctionnement dans l'état de la technique. Ainsi, une infirmière a la possibilité de ne pas utiliser la valve à seuil pour continuer à travailler classiquement avec le dispositif multiaccès de l'invention. Pour utiliser la ligne de rinçage ou d'hydratation avec la valve à seuil, l'infirmière doit couper cette ligne de contournement, par exemple au moyen d'une pince.

En outre, l'ouverture de cette ligne de contournement permet également de réaliser une purge du dispositif par l'administration, avant la liaison avec la pompe, de liquide de rinçage ou d'hydratation à travers le raccord multiaccès et la tubulure inférieure.

Selon un mode de réalisation, ladite tubulure inférieure comporte une chambre compte-gouttes destinée à piéger les bulles d'air présentes dans le liquide circulant dans ladite tubulure inférieure. Ce mode de réalisation permet d'éviter toute incorporation d'air dans l'organisme du patient, facteur pouvant détériorer l'efficacité du traitement.

En outre, la purge réalisée par la ligne de contournement ne permet pas toujours d'extraire l'air contenu au niveau de la valve unidirectionnelle à seuil entre les raccords reliant la ligne de contournement sur la ligne de rinçage ou d'hydratation. Cette chambre compte-gouttes permet également de piéger l'air pouvant rester dans cette partie de la ligne de rinçage ou d'hydratation après la purge.

Selon un mode de réalisation, le dispositif comporte également une ligne de traitement disposée entre l'au moins un raccord multiaccès et ledit au moins un second connecteur supérieur.

De préférence, cette ligne de traitement comporte un filtre sans évent dont le rôle est de filtrer les impuretés restantes dans le produit de traitement. Un filtre classique comporte un évent pour diminuer la pression dans la charge filtrante lorsque le produit à filtrer contient une grande quantité de particules. Les filtres sans évent sont plus rares mais ils permettent d'éviter que la dépression générée par la pompe pour ouvrir la valve unidirectionnelle à seuil ne risque d'ouvrir l'évent du filtre.

La forme et le nombre de raccord multiaccès n'est pas limitatif. L'invention peut être utilisée avec un ou plusieurs raccords multiaccès en forme de Y destinés à relier deux ou trois tubulures. Par exemple, le dispositif peut être configuré pour raccorder de 1 à 6 produits de traitement.

Selon un second aspect, l'invention concerne un système de traitement chimiothérapique comportant :
- un dispositif multiaccès de raccordement à une pompe de perfusion intraveineuse et/ou entérale selon le premier aspect de l'invention ;
- une poche contenant un produit de rinçage reliée sur ledit premier connecteur supérieur ;
- au moins une poche contenant au moins un produit de chimiothérapie reliée sur ledit au moins un second connecteur supérieur ; et
- une pompe connectée sur ladite tubulure inférieure ; ladite pompe étant configurée pour être réglée à débit constant afin d'injecter une quantité de produit de chimiothérapie depuis ladite au moins une poche contenant ledit produit de chimiothérapie ainsi qu'une fraction prédéterminée de cette quantité depuis ladite poche contenant ledit produit de rinçage.

Selon un troisième aspect, l'invention concerne un système de nutrition comportant :
- un dispositif multiaccès de raccordement à une pompe de perfusion intraveineuse et/ou entérale selon le premier aspect de l'invention ;
- une poche contenant un produit d'hydratation reliée sur ledit premier connecteur supérieur ;
- au moins une poche contenant au moins un produit nutritif reliée sur ledit au moins un second connecteur supérieur ; et
- une pompe connectée sur ladite tubulure inférieure ; ladite pompe étant configurée pour être réglée à débit constant afin d'injecter une quantité de produit nutritif depuis ladite au moins une poche contenant ledit produit nutritif ainsi qu'une fraction prédéterminée de cette quantité depuis ladite poche contenant ledit produit d'hydratation.

### Description sommaire des figures

La manière de réaliser l'invention, ainsi que les avantages qui en découlent, ressortiront bien de la description des modes de réalisation qui suivent, donnés à titre indicatif mais non limitatif, à l'appui des figures annexées dans lesquelles les figures 1 à 6 représentent :
[Fig 1] la figure 1 est une représentation schématique d'un système de traitement chimiothérapique selon un premier mode de réalisation ;
[Fig 2] la figure 2 est une représentation schématique d'un système de traitement chimiothérapique selon un second mode de réalisation ;
[Fig 3] la figure 3 est une représentation schématique d'un système de nutrition selon un premier mode de réalisation ;
[Fig 4] la figure 4 est une représentation schématique d'un système de nutrition selon un second mode de réalisation ;
[Fig 5] la figure 5 est une vue en coupe du disque et de la membrane de la valve unidirectionnelle à seuil de la figure 1 dans la position de blocage ; et
[Fig 6] la figure 6 est une vue en coupe du disque et de la membrane de la valve unidirectionnelle à seuil de la figure 1 dans la position de circulation.

### Description détaillée

Dans la suite de la description, les termes supérieur et inférieur font référence à la position des différents éléments lorsqu'ils sont montés sur une ligne de perfusion classique d'un patient. En effet, de manière classique, un pied à perfusion est utilisé pour surélever les poches de perfusion au-dessus du patient lors du traitement car les traitements sont souvent réalisés par gravité. Dans le cadre de l'invention, l'administration de la perfusion est réalisée par une pompe **11** intraveineuse et/ou entérale et il est possible de disposer les poches **19a**, **19b**, **20a**, **20b** au même niveau ou au-dessous du patient. Par exemple, il est possible d'intégrer les poches et la pompe dans un sac à dos afin que le patient puisse recevoir l'administration de son traitement tout en étant mobile.

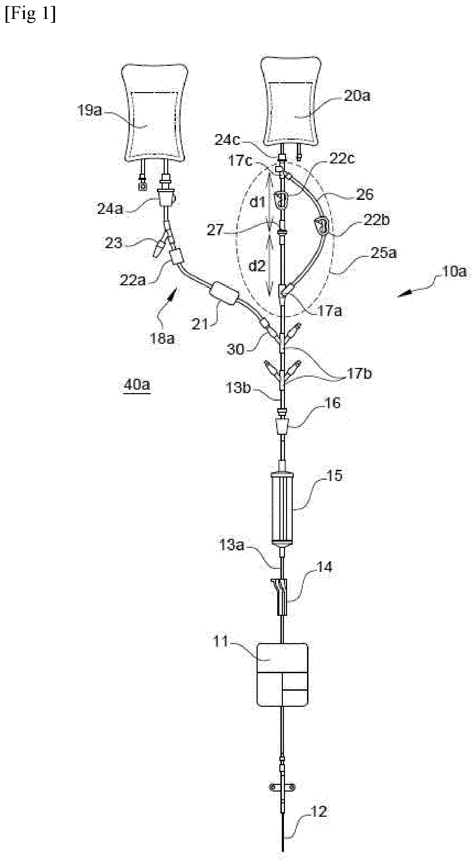

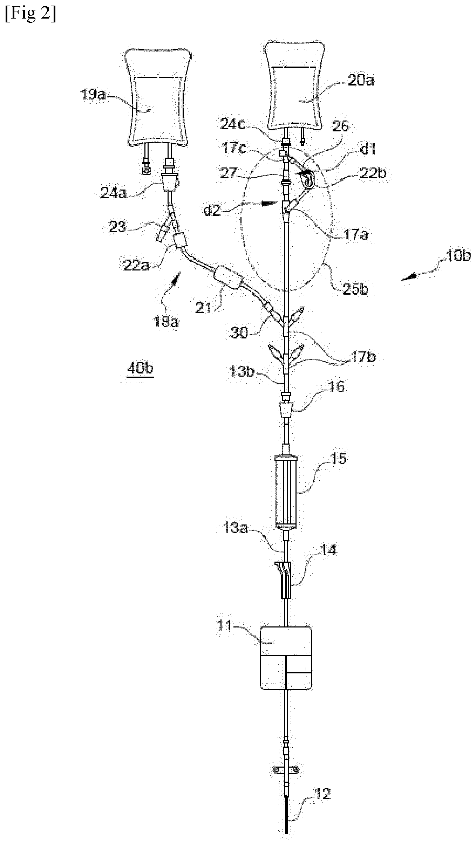

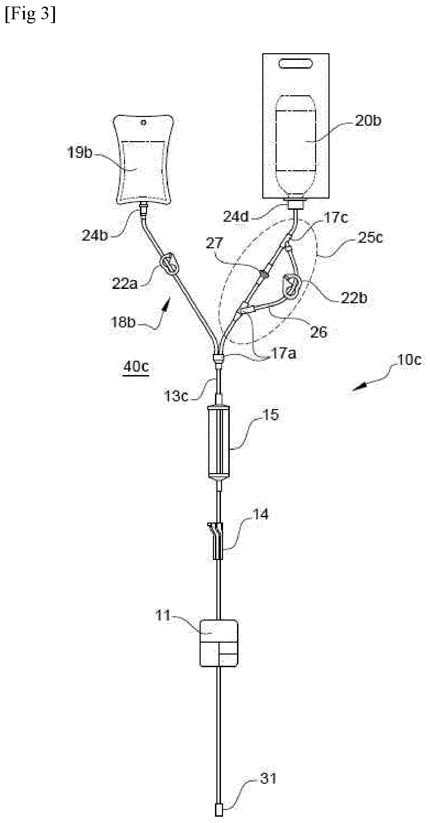

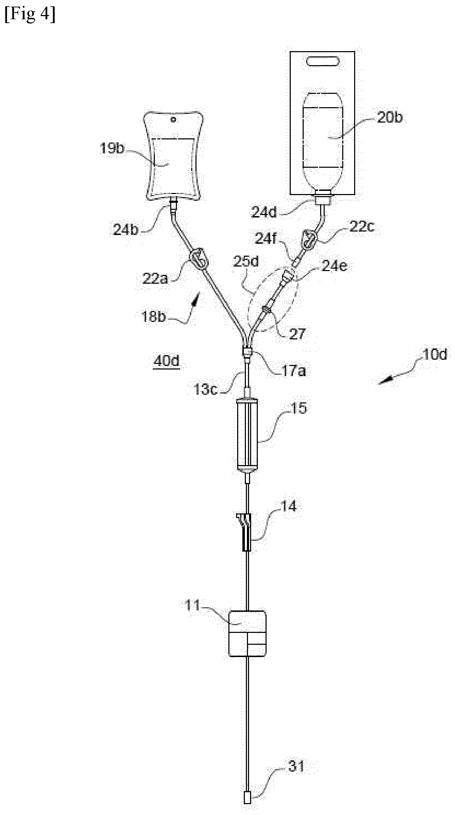

Sur les figures 1 à 4, deux types de système de traitement sont illustrés : deux systèmes de traitement chimiothérapique **40a**, **40b**, figures 1 et 2, et deux systèmes de nutrition **40c**, **40d**, figures 3 et 4.

La figure 1 décrit un système de traitement chimiothérapique **40a** dans le cadre d'une chimiothérapie par voie parentérale. L'administration d'un produit de traitement de chimiothérapie contenu dans une poche **19a** et l'administration d'un produit de rinçage contenu dans une poche **20a** est automatisée au moyen d'une pompe **11.** La sortie de la pompe **11** est reliée par une tubulure à une seringue **12** destinée pénétrer un patient jusqu'à atteindre son réseau sanguin.

Au-dessus de la pompe **11**, le système **40a** comporte un régulateur de débit **14** permettant de régler le débit d'une ligne de perfusion fonctionnant par gravité en cas de dysfonctionnement de la pompe **11.** Une chambre compte-gouttes **15** est disposée au-dessus de ce régulateur de débit **14** et permet de piéger les bulles d'air.

Dans l'exemple des figures 1 et 2, la tubulure inférieure de la pompe **11** est divisée en deux parties : une première partie **13a** supportant le régulateur de débit **14** et la chambre compte-gouttes **15** et une seconde partie **13b** reliant la première partie **13a** à deux raccords **17b.** Les deux parties **13a**, **13b** sont reliées par un site de connexion **16** permettant d'adapter le diamètre des tubulures entre les deux parties **13a**, **13b**. L'invention concerne plus particulièrement un dispositif multiaccès **10a-10d** qui comporte une tubulure inférieure **13a-13c.** En pratique, la tubulure inférieure de l'invention peut correspondre uniquement à la seconde partie **13b**, à l'ensemble des deux parties **13a** et **13b** ou à une tubulure continue **13c** reliant au moins un raccord à la pompe **11**, tel qu'illustré sur les figures 3 et 4.

L'invention peut être utilisée avec un ou plusieurs raccords multiaccès en forme de Y destinés à relier deux ou trois tubulures. Dans l'exemple des figures 1 et 2, deux raccords en Y trois voies **17b** sont superposés de sorte à permettre la connexion d'un produit de rinçage et de quatre produits de traitement. Dans l'exemple des figures 3 et 4, un seul raccord en Y deux voies **17a** est utilisé de sorte à permettre la connexion d'un produit d'hydratationet d'un seul produit de traitement. En variante, trois raccords en Y trois voies **17b** peuvent être utilisés pour connecter six produits de traitement.

Les raccords trois voies **17b** des figures 1 et 2 présentent une tubulure centrale reliée à deux sites de connexion **30** disposés de part et d'autre de la tubulure centrale et connectés hydrauliquement sur cette tubulure.

Au sens de l'invention, un connecteur supérieur du dispositif multiaccès peut correspondre à un site de connexion **30.** En variante, le dispositif multiaccès peut comprendre tout ou partie d'une ligne de traitement **18a-18b.**

En effet, le site de connexion **30** est destiné à être relié à une poche **19a** contenant un produit de traitement tandis que la tubulure centrale forme une ligne de rinçage **25a-25b** reliée à une poche **20a** contenant un produit de rinçage.

Dans l'exemple des figures 1 à 3, la ligne de rinçage ou d'hydratation **25a-25c** s'étend jusqu'à un connecteur **24c-24d** monté sur une sortie d'une poche **20a-20b** contenant un liquide de rinçage ou d'hydratation. En variante, tel qu'illustré sur la figure 4, la ligne d'hydratation **25d** peut présenter deux parties reliées entre elles par deux connecteurs **24e** et **24f.** Au sens de l'invention, la ligne d'hydratation peut s'étendre uniquement jusqu'au connecteur **24e.**

Dans l'exemple des figures 1 et 2, la poche **20a** contient directement le liquide alors que les figures 3 et 4 illustrent une variante dans laquelle la poche **20b** intègre une bouteille dont le goulot est connecté sur la ligne d'hydratation **25c**, **25d**.

Selon l'invention, la ligne de rinçage **25a** comporte une valve unidirectionnelle à seuil **27.**

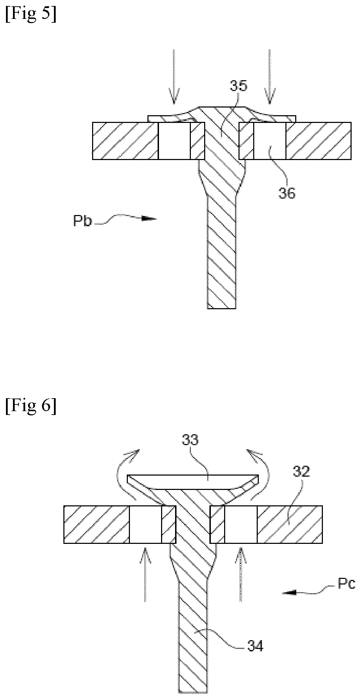

Tel qu'illustré sur les figures 5 et 6, cette valve unidirectionnelle à seuil **27** présente une membrane **33** mobile entre deux positions : une position de blocage **Pb** dans laquelle la circulation du liquide est bloquée dans la ligne de rinçage **25a** au niveau de la valve unidirectionnelle à seuil **27** ; et une position de circulation **Pc** dans laquelle le liquide peut s'écouler dans la ligne de rinçage **25a** à travers la valve unidirectionnelle à seuil **27.** Pour ce faire, la valve **27** comporte un corps intégrant un disque **32** et une membrane **33.** Le disque **32** présente une ouverture cylindrique centrale **35** et au moins une ouverture périphérique **36.** Par exemple, la valve **27** peut présenter deux ouvertures périphériques **36** en forme de croissant.

Dans l'exemple des figures 5 et 6, la valve **27** correspond à une valve de type parapluie. Bien entendu d'autres types de valves unidirectionnelle sont utilisables. Dans la valve **27** de type parapluie des figures 5 et 6, la membrane **33** est maintenue par un manche **34** fixé dans l'ouverture centrale **35** du disque **32.** Cette membrane **33** présente une forme circulaire dont les extrémités radiales recouvrent l'au moins une ouverture périphérique **36.** Ainsi, dans la position de blocage **Pb** illustrée sur la figure 5, la pression exercée par le fluide sur les extrémités radiales de la membrane **33** maintient l'obturation de l'au moins une ouverture périphérique **36** par la membrane **33.** Au contraire, dans la position de circulation **Pc** illustrée sur la figure 6, la pression exercée par le fluide depuis l'au moins une ouverture périphérique **36** entraîne une déformation des extrémités radiales de la membrane **33** de sorte à ménager un passage pour le fluide autour de ses extrémités radiales. La forme biseautée de la membrane **33** déplace les extrémités radiales dans la position de blocage **Pb** lorsque la pression exercée depuis l'au moins une ouverture périphérique **36** est insuffisante. La forme biseautée de la membrane **33** forme donc des moyens de rappel de la valve **27** dans la position de blocage **Pb.**

Ainsi, la valve **27** est configurée de sorte à s'ouvrir lorsqu'une dépression est formée par la pompe **11** sur la tubulure inférieure **13a**, **13b.** De préférence, le seuil d'ouverture de la valve **27** est compris entre 100 et 400 mBar, préférentiellement entre 150 et 250 mBar.Dans les modes de réalisation des figures 1 et 3, la ligne de rinçage ou d'hydratation **25a-25c** comporte également une ligne de contournement **26**, également appelée *by-pass* dans la littérature anglo-saxonne. Cette ligne de contournement **26** est connectée sur la ligne de rinçage ou d'hydratation **25a-25c** au moyen de deux raccords en Y deux voies **17a** et **17c.** Le raccord **17c** est disposé au-dessus de la valve **27** alors que le raccord **17a** est disposé au-dessous de la valve **27.** La distance **d1** entre la valve **27** et le raccord **17c** est sensiblement équivalente à la distance **d2** entre la valve **27** et le raccord **17a.** Dans l'exemple de la figure 1, cette distance **d1**, **d2** est comprise entre 5 et 10 cm.

Pour limiter la quantité d'air restant dans la ligne de rinçage ou d'hydratation **25a-25c** après une purge effectuée par la ligne de contournement **26**, le mode de réalisation de la figure 2 propose de réduire cette distance **d1**, **d2.** Ainsi, dans le mode de réalisation de la figure 2, cette distance **d1**, **d2** est comprise entre 1 et 3 cm. Dans le mode de réalisation de la figure 3, cette distance est comprise entre 3 et 5 cm. En variante, dans la mode de réalisation de la figure 4, aucune ligne de contournement n'est utilisée.

La chambre compte-gouttes **15** est configurée de sorte à pouvoir absorber l'air ne pouvant être purgé par la ligne de contournement, c'est-à-dire l'air présent dans les tubulures au niveau de ces distances **d1**, **d2**. Par exemple, pour une tubulure d'une section de 2.5 mm et une distance **d1**, **d2** de 5cm, la chambre compte-gouttes **15** doit pouvoir absorber un volume d'air d'environ 0.5 cm³.

La longueur de la ligne de contournement **26** permet de faciliter le positionnement d'une pince **22b** lorsqu'une infirmière veut terminer la purge. Dans le mode de réalisation de la figure 4, c'est-à-dire sans ligne de contournement **26**, la pince **22b** peut être disposée directement au-dessus de la valve **27.**

En outre, en utilisant une longueur suffisante entre la valve **27** et le raccord **17c**, il est possible de positionner une pince **22c** pour former une sécurité supplémentaire dans le cas où la valve présenterait un dysfonctionnement.

Outre la ligne de rinçage **25a-25d**, l'administration du produit de traitement est assurée par une ligne de traitement **18a-18b** reliant une poche **19a-19b** contenant le produit de traitement sur le raccord multiaccès **17a-17b.**

Dans l'exemple des figures 1 et 2, la ligne de traitement **18a** s'étend jusqu'à un connecteur **24a** monté sur une sortie d'une poche **19a** contenant un produit de traitement chimiothérapique. La ligne de traitement **18a** comprend un filtre sans évent **21**, qui est relié directement au connecteur **30** du raccord multiaccès **17b.** Un site d'injection **23** est disposé entre le filtre sans évent **21** et le connecteur 24**a**, de sorte à permettre l'injection du produit de traitement chimiothérapique dans la poche **19a** lors de la préparation dans la ligne de traitement **18a.** Si l'infirmière veut interrompre l'acheminement du produit de traitement à la pompe, une pince **22a** peut être fixée sur la ligne de traitement **18a**, par exemple entre le site d'injection **23** et le filtre sans évent **21.**

Selon un autre mode de réalisation illustré sur les figures 3 et 4, une ligne de traitement **18b** s'étend entre un raccord **17a** et un connecteur **24b** monté sur une sortie d'une poche **19b** contenant un produit de nutrition. Cette ligne de traitement **18b** comporte uniquement une pince **22a** qui peut être fixée sur la ligne de traitement **18b** si l'infirmière veut interrompre l'acheminement du produit de traitement à la pompe.

Pour réaliser un protocole thérapeutique, une première étape consiste généralement à réaliser une purge des tubulures de la ligne de rinçage ou d'hydratation **25a-25d** et de la tubulure inférieure **13a-13c.** Par exemple, cette purge peut être effectuée en retirant la pince **22b** de la ligne de contournement 26 et en activant la pompe pendant quelques secondes. Suite à cette purge, la pince **22b** est repositionnée sur la ligne de contournement **26.** De préférence, la ligne de traitement **18a-18b** est connectée après la purge. Dans le cas des figures 3 et 4, la pince **22a** est positionnée au plus proche du raccord **17a** lorsqu'une purge est réalisée.

Suite à cette phase de purge, une infirmière peut lancer la thérapie en retirant les pinces **22a** et **22c** d'une seule ligne de traitement **18a-18b** et de la ligne de rinçage ou d'hydratation **25a-25d.** L'infirmière active ensuite la pompe **11** avec un débit constant. Le temps d'activation de la pompe **11** est déterminé en fonction de la quantité de produit présent dans la poche **19a-19b** et de la quantité souhaitée de produit de rinçage ou d'hydratation.

Lorsque la poche **19a-19b** contenant le produit de traitement est vide, la dépression générée par la pompe **11** ouvre automatiquement la valve **27.** Le système **40a-40d** procède alors à l'administration du produit de rinçage ou d'hydratation.

La pompe **11** s'arrête automatiquement à la fin de l'administration du produit de rinçage ou d'hydratation lorsque la quantité souhaitée de produit de rinçage ou d'hydratation a été injectée. Ainsi, il n'est pas nécessaire d'avertir l'infirmière peu avant la fin d'un traitement thérapeutique, afin que l'infirmière soit présente pour préparer la ligne de rinçage ou d'hydratation.

L'invention permet ainsi d'automatiser la transition entre l'injection du produit de traitement et du produit de rinçage ou d'hydratation, ce qui permet d'optimiser le temps de présence des professionnels de la santé, tout en préservant l'efficacité du traitement du patient.

## Revendications

1. Dispositif multiaccès de raccordement (10a-10d) à une pompe de perfusion (11) intraveineuse et/ou entérale, ledit dispositif comportant :
- un premier connecteur supérieur (24c-24d) destiné à acheminer un produit de rinçage ou d'hydratation ;
- au moins un second connecteur supérieur (24a-24b, 30) destiné à acheminer au moins un produit de traitement ;
- une tubulure inférieure (13a-13c) destinée à être connectée à une pompe (11) de perfusion ; et
- au moins un raccord multiaccès (17a-17b) mettant en connexion fluidique les connecteurs supérieurs (24a-24b, 30) et ladite tubulure inférieure (13a-13c) ; ***caractérisé en ce que*** le dispositif comporte également une ligne de rinçage ou d'hydratation(25a-25d) disposée entre l'au moins un raccord multiaccès (17a-17b) et ledit premier connecteur supérieur (24c-24d) ;
ladite ligne de rinçage ou d'hydratation (25a-25d) comportant une valve unidirectionnelle à seuil (27) intégrant :
une membrane (33) mobile entre deux positions :une position de blocage (Pb) dans laquelle la circulation du liquide est bloquée dans ladite ligne de rinçage ou d'hydratation (25a-25d) au niveau de ladite valve unidirectionnelle à seuil(27) ; et une position de circulation (Pc) dans laquelle le liquide peut s'écouler dans ladite ligne de rinçage ou d'hydratation (25a-25d) à travers ladite valve unidirectionnelle à seuil (27) ; et
des moyens de rappel de ladite membrane (33) dans ladite position de blocage (Pb) ; ladite valve unidirectionnelle à seuil (27) étant montée sur ladite ligne de rinçage ou d'hydratation (25a-25d) de sorte qu'une dépression formée sur ladite tubulure inférieure(13a-13c) entraîne un déplacement de ladite membrane (33) entre ladite position de blocage (Pb) et ladite position de circulation (Pc),
ladite valve unidirectionnelle à seuil (27) étant destinée à coopérer avec ladite pompe de perfusion (11) intraveineuse et/ou entérale de sorte que la dépression formée par ladite pompe de perfusion (11), lorsque la première poche est vidée, entraîne automatiquement l'ouverture de ladite valve unidirectionnelle à seuil (27) et l'approvisionnement en liquide de rinçage ou d'hydratation de ladite pompe de perfusion (11).

2. Dispositif multiaccès selon la revendication 1, ***caractérisé en ce que*** ladite valve unidirectionnelle à seuil (27) présente un seuil d'ouverture compris entre 100 et 400 mBar.

3. Dispositif multiaccès selon la revendication 2, ***caractérisé en ce que*** ladite valve unidirectionnelle à seuil (27) présente un seuil d'ouverture compris entre 150 et 250 mBar.

4. Dispositif multiaccès selon l'une des revendications 1 à 3, ***caractérisé en ce que*** ladite ligne de rinçage ou d'hydratation (25a-25b) comporte deux raccords multiaccès (17a, 17c) disposés au-dessus et en-dessous de ladite valve unidirectionnelle à seuil (27) et reliés par une tubulure formant une ligne de contournement (26) de ladite valve unidirectionnelle à seuil (27).

5. Dispositif multiaccès selon l'une des revendications précédentes, ***caractérisé en ce que*** ladite tubulure inférieure (13a-13c) comporte une chambre compte-gouttes (15) destinée à piéger les bulles d'air présentes dans le liquide circulant dans ladite tubulure inférieure (13a-13c).

6. Dispositif multiaccès selon l'une des revendications précédentes, ***caractérisé en ce que*** le dispositif comporte également une ligne de traitement (18a-18b) disposée entre l'au moins un raccord multiaccès (17a-17b) et ledit au moins un second connecteur supérieur (24a-24b).

7. Dispositif multiaccès selon l'une des revendications précédentes, ***caractérisé en ce que*** ledit au moins un raccord multiaccès (17a-17b) permet de raccorder de 1 à 6 produits de traitement.

8. Système de traitement chimiothérapique (40a-40b) comportant :
- un dispositif multiaccès (10a-10d) selon l'une des revendications 1 à 7 ;
- une poche (20a) contenant un produit de rinçage reliée sur ledit premier connecteur supérieur (24c-24d) ;
- au moins une poche (19a) contenant au moins un produit de chimiothérapie reliée sur ledit au moins un second connecteur supérieur (24a-24b, 30) ; et
- une pompe (11) connectée sur ladite tubulure inférieure(13a-13b) ; ladite pompe (11) étant configurée pour être réglée à débit constant afin d'injecter une quantité de produit de chimiothérapie depuis ladite au moins une poche (19a) contenant ledit produit de chimiothérapie ainsi qu'une fraction prédéterminée de cette quantité depuis ladite poche (20a) contenant ledit produit de rinçage.

9. Système de nutrition (40c-40d) comportant :
- un dispositif multiaccès (10a-10d) selon l'une des revendications 1 à 7 ;
- une poche (20b) contenant un produit d'hydratation reliée sur ledit premier connecteur supérieur (24c-24d) ;
- au moins une poche (19b) contenant au moins un produit nutritif reliée sur ledit au moins un second connecteur supérieur (24a-24b, 30) ; et
- une pompe (11) connectée sur ladite tubulure inférieure (13c) ; ladite pompe (11) étant configurée pour être réglée à débit constant afin d'injecter une quantité de produit nutritif depuis ladite au moins une poche (19b) contenant ledit produit nutritif ainsi qu'une fraction prédéterminée de cette quantité depuis ladite poche (20b) contenant ledit produit d'hydratation.
